# EUROPÄISCHE PATENTANMELDUNG

(11) **EP 1 403 807 A1**
(43) Veröffentlichungstag der Anmeldung: **31.03.2004**
(21) Anmeldenummer: 02021476.3
(22) Anmeldetag: 25.09.2002
(51) Int. Cl.: G06F 19/00

(54) **Patientenüberwachungssystem zur automatischen Erfassung der Einschränkungen von Alltagsfähigkeiten**

(71) Anmelder: SIEMENS AKTIENGESELLSCHAFT, 80333 München (DE)
(72) Erfinder: Abraham-Fuchs, Klaus, 91058 Erlangen (DE); Bieger, Johannes, Dr., 80638 München (DE); Hengerer, Arne, Dr., 91052 Erlangen (DE); Rumpel, Eva, Dr., 91052 Erlangen (DE); Tietze, Daniel, 91080 Spardorf (DE)

(57) **Zusammenfassung**

Patientenüberwachungssystem zur Erfassung der Einschränkungen von Alltagsfähigkeiten mithilfe eines eine Vielzahl von Fragen umfassenden Fragenkatalogs, mit einem Expertensystem, das über elektronische Übertragungswege wie Telefon, SMS, E-Mail oder dergleichen, jedem zu betreuenden Patienten automatisch und regelmäßig seine persönlichen Verhältnisse und/oder seine Krankengeschichte berücksichtigende einzelne Fragen oder Unterkombinationen des Fragenkatalogs vorlegt und die Antworten dokumentiert und auswertet und daraus gegebenenfalls neue spezifische Fragen an den Patienten ableitet.

## Beschreibung

Die Erfindung bezieht sich auf ein Patientenüberwachungssystem zur Erfassung der Einschränkungen von Alltagsfähigkeiten mithilfe eines eine Vielzahl von Fragen umfassenden Fragenkatalogs.

Zunehmend werden Patienten mit Hilfe von telemedizinischen Versorgungsformen betreut, z. B. beim Management chronischer Krankheiten (z. B. Diabetes, Asthma, Alzheimer, Depression), in der Nachsorge nach stationärer Rehabilitation (z. B Schlaganfall), oder zur Begleitung pflegebedürftiger Personen. Um die Entscheidungsfindung des betreuenden medizinischen Fachpersonals zu unterstützen, werden medizinisch relevante Daten (z. B. Blutdruck, Blutzuckerwerte, EKG) beim Patienten zu Hause erfasst und mit Datenfernübertragung an den Arzt übermittelt.

Zur Beurteilung, wie gut der Patient mit seiner Krankheit im Alltag zu Hause zurecht kommt, sind jedoch nicht nur physiologische Messwerte wichtig, sondern auch das Wissen über die Qualität, mit der der Patient wichtige und typische Verrichtungen des täglichen Lebens ausführen kann. Solche Fragen sind zum Beispiel:
- können Sie Ihre Treppe zum ersten Stock ohne Beschwerden hinaufgehen?
- hatten Sie heute Probleme beim Ankleiden?
- kommen Sie mit dem Duschen zurecht?
- hatten Sie Probleme mit dem Einkauf notwendiger Lebensmittel?

Solche Fragebogen, die die Qualität erfassen, mit der die Patienten diese typischen "Activities of Daily Living" ausführen können, sind in verschiedenen Ausführungen bereits als Standard vorhanden und umfassen typischerweise ca. 15 bis 50 Fragen. Sie werden meist als Fragebögen zur "Erfassung der Lebensqualität" ("Quality of Living") bezeichnet, und werden typischerweise im Rahmen von klinischen Studien eingesetzt. Dies geschieht allerdings meist nur episodisch in großen Zeitintervallen, etwa zum Anfang und zum Abschluss einer klinischen Studie. Alle zur Erfassung der Lebensqualität erforderlichen Fragen können auch bei einer solchen klinischen Studie nicht gestellt werden, da insgesamt hierfür Hunderte von Fragen gestellt werden könnten, aus denen spezifische Fragebögen ja nach Art des Leidens immer nur eine Gruppe von maximal fünfzig Fragen herausgreifen. Wünschenswert für die Beurteilung des Zustandes eines Patienten wäre es aber sicher, wenn - wenn auch gegebenenfalls nur ab und zu - auch ganz andere Befindlichkeitsfragen aus dem großen Gesamtfragenkatalog gestellt werden könnten.

Im Falle einer telemedizinischen Betreuung wäre es jedoch von Vorteil, wenn auch die Entwicklung dieser Alltagsfähigkeiten, besonders mit Hinblick auf die frühzeitige Erkennung einer Verschlechterung, kontinuierlich überwacht werden könnte ("Monitoring der Lebensqualität"). Hierzu ist es jedoch nicht möglich, täglich oder auch nur wöchentlich vom Patienten den ganzen Fragebogen beantworten zu lassen, da der Patient diesen Arbeitsaufwand nicht akzeptieren würde. Genauso ist es aus Kostengründen unmöglich, die Erfassung dieser Information interaktiv mit medizinischem Betreuungspersonal durchzuführen.

Bisher versuchte man das Problem durch wiederholte Besuche beim und Beurteilung durch den Therapeuten zu lösen oder aber auch aufgrund einer Selbsteinschätzung des Patienten, der mittels eines Fragebogens zu Lebensqualität oder durch das Führen eines Patiententagebuches erfasst wurde, oder - in seltenen Fällen - mit aufwändiger Sensorik. Die Selbsteinschätzung des Patienten ist zur Datenerhebung jedoch kaum geeignet, da die Patienten (insbesondere solche mit neurologischen bzw. psychiatrischen Problemen) sich selbst nicht objektiv wahrnehmen können. Patiententagebücher werden erfahrungsgemäß nicht zuverlässig über einen ausgedehnten Zeitraum hinweg geführt. Typische sensorische Lösungen sind technologisch und in der Benutzung aufwändig und können nur ein beschränktes Bild liefern, da sie üblicherweise nur die Einschränkung selbst messen, z. B. die Beweglichkeit von Gelenken, die Reaktionszeiten auf bestimmte Ereignisse usw. und etwa vorhandene Kompensationsmechanismen außer acht lassen. Folglich stehen zur Beurteilung eines Therapieerfolges nur selten verlässliche Datenbasen zur Verfügung. In den meisten Fällen findet nach der Entlassung des Patienten aus der Therapie überhaupt keine Datenerhebung statt, weil der damit verbundene Kostenaufwand bei den heute verwendeten Methoden zu hoch ist.

Aus der DE 196 37 383 A1 ist bereits eine Datenerfassungsund Auswertevorrichtung bekannt geworden, bei der Zustand einer Person durch Sensoren erfasst und über eine Auswerteeinheit zur Beurteilung der Situation dieser Personen im Vergleich zu ihrem gewohnten Tagesablauf unter Berücksichtigung weiterer Daten wie z. B. der Uhrzeit und des Aufenthaltsorts ausgewertet wird. Ein solches Sensorerfassungs- und Auswertesystem berücksichtigt aber gerade nicht die häufig nicht sensorisch greifbaren Einschränkungen von Alltagsfähigkeiten, wie beispielsweise
- Hatten Sie Schmerzen?
- Haben Sie schlecht geschlafen?
- Fühlen Sie sich schwach?
- Fehlt Ihnen der Appetit?
- Hatten Sie Stuhlgang, gegebenenfalls haben Sie Durchfall?
- Haben Sie Schwierigkeiten beim Zeitung lesen?
usw.

Gerade diese Einschränkung von Alltagsfähigkeiten sind es aber, die die wesentlichen Ansätze zu einer Therapie und gegebenenfalls auch Änderung einer Therapie geben können.

Der Erfindung liegt daher die Aufgabe zugrunde, ein Patientenüberwachungssystem zur automatischen Erfassung der Einschränkungen von Alltagstätigkeiten mithilfe eines eine Vielzahl von Fragen umfassenden Fragenkatalogs zu schaffen, das einfach und zuverlässig arbeitet und den Patienten nicht überfordert, sodass nicht zu befürchten ist, dass er seine Mitarbeit verweigert.

Zur Lösung dieser Aufgabe ist ein solches Patientenüberwachungssystem gekennzeichnet durch ein Expertensystem, das über elektronische Übertragungswege wie Telefon, SMS, E-Mail oder dergleichen, jedem zu betreuenden Patienten automatisch und regelmäßig seine persönlichen Verhältnisse und/oder seine Krankengeschichte berücksichtigende einzelne Fragen oder Unterkombinationen des Fragenkatalogs vorlegt und die Antworten dokumentiert und auswertet und daraus gegebenenfalls neue spezifische Fragen an den Patienten ableitet.

Die so erhobenen Daten sind äußerst hilfreich, um den Therapieerfolg zu beurteilen und um gegebenenfalls gezielt weiterführende Therapiemaßnahmen zu initiieren.

Dabei geht die Erfindung von der Erkenntnis aus, dass es aus medizinischer Sicht nicht notwendig ist, alle Fragen eines Lebensqualitäts-Fragenkatalogs am Stück in einer Sitzung zu beantworten. Ebenso ist das Zeitintervall, in dem bestimmte Fragen neu gestellt werden müssen, weil sich die Situation des Patienten geändert haben könnte, eventuell sehr unterschiedlich. Die Erfindung geht also davon aus, dass der medizinisch sinnvolle Informationsgewinn auch dadurch zu erzielen ist, dass z. B. jeden Tag nur eine oder zwei Fragen gestellt werden, oder auch nur jeden zweiten oder dritten Tag, und die Fragen auch nicht mit gleicher Häufigkeit und in einer festen Reihenfolge gestellt werden sollen. Vielmehr sollten Reihenfolge und Häufigkeit der Fragen individualisiert an die Krankengeschichte und aktuelle Situation angepasst werden, bzw. aus den Antworten auf die zuletzt gestellten Fragen abgeleitet werden.

Aus diesem Grund ist es zweckmäßig, das Patientenüberwachungssystem so auszugestalten, dass für jeden Patienten ein seine persönlichen Verhältnisse und seine Krankengeschichte berücksichtigender Fragenkatalog erstellt wird, wozu es wiederum zweckmäßig ist, dass das Expertensystem Zugriff zu einer zentralen oder dezentralen elektronischen Patientenakte sowie auch zu den Sensordaten aus einem Patientenmonitoring hat.

Da auch äußere Einflussfaktoren für das Wohlbefinden und die Alltagsfähigkeiten eines Patienten großen Einfluss haben können, soll in Ausgestaltung der Erfindung das Expertensystem mit Datenbanken über bekannte Einflussfaktoren, wie z. B. Wetter, Pollenflugbericht oder dergleichen, verbunden sein.

Zweckmäßigerweise lässt sich ein erfindungsgemäßes Patientenüberwachungssystem in ein automatisiertes Call-Center integrieren.

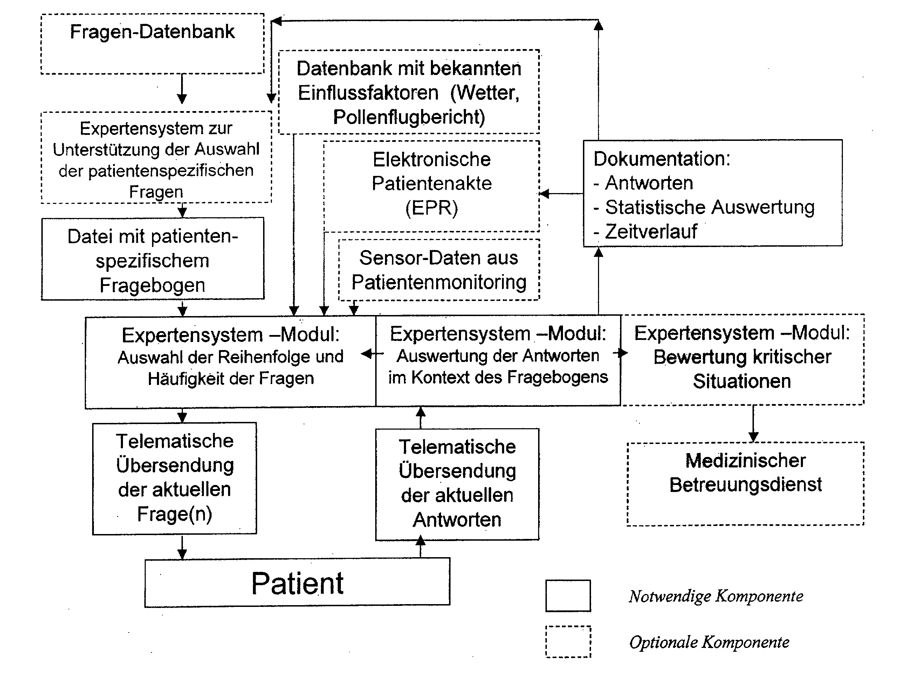

Weitere Vorteile, Merkmale und Einzelheiten der Erfindung ergeben sich aus der nachfolgenden Beschreibung eines Ausführungsbeispiels sowie anhand der Zeichnung, die ein Ablaufdiagramm des Patientenüberwachungssystems darstellt.

Für jeden Patienten wird ein individualisierter Fragenkatalog in elektronischer Form (z. B. Datei, Datenbank, Datenbankabfrage) zusammengestellt, entweder durch neue Erstellung oder durch Auswahl aus einer geeigneten Fragendatenbank. Diese Auswahl aus einer umfassenden Fragendatenbank erfolgt dabei sinnvoller weise über ein Expertensystem, das anhand der ihm zur Verfügung gestellten spezifischen Daten eines Patienten, einschließlich seiner Krankenvorgeschichte, eine sinnvolle Auswahl eines gegebenenfalls 10 bis 50 oder mehr Fragen umfassenden Fragenkatalogs erstellt, der praktisch alle für den jeweiligen Patienten und die Beurteilung der Einschränkungen seiner Alltagsfähigkeiten notwendigen und aussagekräftigen Fragen beinhaltet.

Ein computergestütztes Expertensystem wählt nun aus dem elektronischen patientenspezifischen Fragenkatalog unter Berücksichtigung von Regeln für die zeitliche Abfolge, mit der diese Fragen gestellt werden und gegebenenfalls weiterer Informationen aus einer eventuell vorhandenen elektronischen Patientenakte des Patienten eine, oder gegebenenfalls auch mehrere, Frage(n) aus, die dem Patienten an diesem Tag gestellt werden sollen. Diese Regeln können statisch (Frage k am n-ten Tag eines Fragezyklus) oder dynamisch gesteuert sein (z. B. wenn Frage x mit ja beantwortet wird, stelle Frage y am darauffolgenden Tag, fahre dann mit dem statischen Katalog fort). Die dynamischen Regeln können auch durch zusätzlich gewonnene Daten, wie z. B. durch telemedizinisch erhobene Blutdruckwerte, gesteuert werden.

Die ausgewählte Frage wird mit elektronischen Hilfsmitteln an den Patienten übermittelt, z. B. Telefon, SMS, E-Mail oder dergleichen und der Patient zur Beantwortung der Frage aufgefordert. Die Antwort sollte vorzugsweise nur aus ja, nein oder weiß nicht oder allenfalls einer Benotung auf einer Skala, z. B. 1 bis 3 oder 0 bis 5, bestehen. Die Antwort wird von Patienten über Telefontastatur eingegeben oder in einer E-Mail-Antwort zurückgesendet.

Optional können auch allgemein bekannte Einflussfaktoren, wie z. B. das Wetter, die Jahreszeit, bekannte Epidemien, Pollenflugbericht usw. Berücksichtigung bei der Auswahl der Fragen, aber auch bei der Auswertung der Antworten auf die Fragen, finden.

Der empfangende Computer des Patientenüberwachungssystems nimmt die Antwort entgegen und trägt sie in eine Patientendatenbank ein, aus der sich unter anderem ein "Lebensqualitäts-Tagebuch" des Patienten erstellen lässt. Zusätzlich dazu kann ein Expertensystem die Antworten auswerten und bei medizinisch kritisch erscheinenden Situationen eine zugeordneten medizinischen Betreuungsdienst benachrichtigen.

Darüber hinaus kann dem Patienten bei einem Telefon-Call-Center die Möglichkeit gegeben werden, durch Drücken einer bestimmten Taste mit einem Berater statt mit dem Call-Center-Computer verbunden zu werden.

Neben der Beantwortung der eben gestellten Fragen hat der Patient auch die Möglichkeit, Zusatzinformationen von sich aus einzugeben, wozu er diese zusätzlichen Informationen vorzugsweise aus vorgegebenen Listen auswählt, sodass sie ebenfalls automatisch verarbeitet werden können.

Während die Antworten auf einzelne Fragen für sich genommen kein fundiertes Bild der Alltagsfähigkeiten erlauben, liefert die angepasste Auswahl und die regelmäßige Erhebung über einen längeren Zeitraum eine relativ aussagekräftige Analyse. Hierbei wird auch dadurch ein höheres Maß an Objektivität erzielt, das viele Parameter indirekt erhoben werden und so der Patient nicht bewusst oder unbewusst manipuliert werden kann. Gegebenenfalls können die Fragen mit sensorischer Datenerhebung, z. B. Beugungssensoren, Computertest kognitiver Fähigkeiten etc., kombiniert und ergänzt werden. Zur umfassenden Auswertung aller gesammelten Daten kann der Benutzer, z. B. der Therapeut, der Disease Manager, die Krankenversicherung oder dergleichen, mittels eines Statistikmoduls Informationen zur Entwicklung der Alltagsfähigkeit der Patienten abfragen.

Schließlich bietet ein kontinuierliches Langzeitmonitoring der Lebensqualität der Patienten in der Reha-Nachsorge die Möglichkeit, den Therapieerfolg und damit die Qualität der Reha-Leistung auf eine objektive und statistisch signifikante Art und Weise quantitativ zu messen.

## Patentansprüche

1. Patientenüberwachungssystem zur Erfassung der Einschränkungen von Alltagsfähigkeiten mithilfe eines eine Vielzahl von Fragen umfassenden Fragenkatalogs, **gekennzeichnet durch** ein Expertensystem, das über elektronische Übertragungswege wie Telefon, SMS, E-Mail oder dergleichen, jedem zu betreuenden Patienten automatisch und regelmäßig seine persönlichen Verhältnisse und/oder seine Krankengeschichte berücksichtigende einzelne Fragen oder Unterkombinationen des Fragenkatalogs vorlegt und die Antworten dokumentiert und auswertet und daraus gegebenenfalls neue spezifische Fragen an den Patienten ableitet.

2. Patientenüberwachungssystem nach Anspruch 1,**dadurch gekennzeichnet, dass** das Expertensystem Zugriff zu einer zentralen oder dezentralen elektronischen Patientenakte (EPR) hat.

3. Patientenüberwachungssystem nach Anspruch 1 oder 2, **dadurch gekennzeichnet, dass** das Expertensystem Zugriff zu den Sensor-Daten aus einem Patientenmonitoring hat.

4. Patientenüberwachungssystem nach einem der Ansprüche 1 bis 3, **dadurch gekennzeichnet, dass** das Expertensystem mit Datenbanken über bekannte Einflussfaktoren, wie z. B. Wetter, Pollenflugbericht oder dergleichen, verbunden ist.

5. Patientenüberwachungssystem nach einem der Ansprüche 1 bis 4, **dadurch gekennzeichnet, dass** es in ein automatisiertes Call-Center integriert ist.

6. Patientenüberwachungssystem nach einem der Ansprüche 1 bis 5, **dadurch gekennzeichnet, dass** für jeden Patienten ein elektronisches "Lebensqualitäts-Tagebuch" erstellt und abgespeichert wird.

7. Patientenüberwachungssystem nach einem der Ansprüche 1 bis 6, **dadurch gekennzeichnet, dass** das Expertensystem bei medizinisch kritisch erscheinenden Situationen einen medizinischen Betreuungsdienst benachrichtigt.

8. Patientenüberwachungssystem nach einem der Ansprüche 6 bis 7, **dadurch gekennzeichnet, dass** die Eingabevorrichtung beim Patienten mit einer Ruftaste versehen ist um ihn statt mit dem Call-Center-Computer mit einem Berater zu verbinden.

9. Patientenüberwachungssystem nach einem der Ansprüche 1 bis 8, **dadurch gekennzeichnet, dass** der Patient, vorzugsweise aus einer vorgegebenen Liste mit automatisch verarbeitbaren Informationen, von sich aus Zusatzinformationen an das Expertensystem zu geben.
